# EUROPEAN PATENT APPLICATION

(11) **EP 4 645 328 A1**
(43) Date of publication of application: **05.11.2025**
(21) Application number: 23911541.3
(22) Date of filing: 01.12.2023
(51) Int. Cl.: G16H 20/60

(54) **INFORMATION PROCESSING SYSTEM, INFORMATION PROCESSING METHOD, AND PROGRAM**

(30) Priority: 30.12.2022 JP 2022212866
(71) Applicant: Shinshu University, Matsumoto-shi, Nagano 390-8621 (JP); Wellnas.Co.,Ltd, Tokyo 166-0003 (JP)
(72) Inventor: NAKAMURA, Kozo, Kamiina-gun, Nagano 399-4598 (JP)
(74) Representative: Winter, Brandl - Partnerschaft mbB
(86) International application number: PCT/JP2023/043153
(87) International publication number: WO 2024/142760

(57) **Abstract**

Optimal food information can be provided to individual users.

An information processing system includes: a prediction model storage unit that stores, for each user, a prediction model that predicts physical data regarding a body of the user based on intake amounts of a plurality of dietary components taken by the user, the prediction model including a degree to which each dietary component improves or deteriorates the physical data; a food database that stores a content of the dietary component contained in a food; and an influence amount calculation unit that calculates an influence amount which is an amount by which the food improves or deteriorates the physical data of the user based on the content of the dietary component included in the food and the degree related to the dietary component.

## Description

### Technical Field

The present invention relates to an information processing system, an information processing method, and a program.

### Background Art

Patent Literature 1 discloses a system that analyzes meal information and vital data and provides advice.

### Citation List

### Patent Literature

Patent Literature 1: JP 2004-302498 A

### Summary of Invention

### Technical Problem

However, in the system described in Patent Literature 1, it is necessary to prepare advice as a database in advance, and thus it is difficult to consider individual differences.

The present invention has been made in view of such a background, and an object of the present invention is to provide optimal food information to individual users.

### Solution to Problem

A main invention of the present invention for solving the above problems is an information processing system including: a prediction model storage unit that stores, for each user, a prediction model that predicts physical data regarding a body of the user based on intake amounts of a plurality of dietary components taken by the user, the prediction model including a degree to which each dietary component improves or deteriorates the physical data; a food database that stores a content of the dietary component contained in a food; and an influence amount calculation unit that calculates an influence amount which is an amount by which the food improves or deteriorates the physical data of the user based on the content of the dietary component included in the food and the degree related to the dietary component.

Other problems disclosed in the present application and methods for solving the problems will be clarified by the sections and drawings of the embodiments of the invention.

### Advantageous Effects of Invention

According to the present invention, it is possible to provide optimal food information to individual users.

### Brief Description of Drawings

Fig. 1 is a diagram illustrating a configuration example of a food information providing system of the present embodiment.
Fig. 2 is a diagram illustrating a hardware configuration example of a server device 2.
Fig. 3 is a diagram illustrating a software configuration example of the server device 2.
Fig. 4 is a diagram illustrating an example of actual value information.
Fig. 5 is a diagram illustrating a flow of determination processing for a contribution amount and an interference amount.
Fig. 6 is a diagram illustrating an output example of an ingredient output unit 221.
Fig. 7 is a diagram illustrating an output example of the ingredient output unit 221.
Fig. 8 is a diagram illustrating an output example of a dish output unit 222.
Fig. 9 is a diagram illustrating an output example of the dish output unit 222.
Fig. 10 is a diagram illustrating an output example of a product output unit 226.
Fig. 11 is a diagram illustrating an output example of the product output unit 226.
Fig. 12 is a diagram illustrating an output example of the ingredient output unit 221.
Fig. 13 is a diagram illustrating an output example of the dish output unit 222.
Fig. 14 is a diagram illustrating an output example of a recipe output unit 223.
Fig. 15 is a diagram illustrating an output example of an explanatory information output unit 224.
Fig. 16 is a diagram illustrating an example of outputting access information by an access information output unit 225.
Fig. 17 is a diagram illustrating an output example of a physical store information output unit 227.
Fig. 18 is a diagram illustrating an output example of the physical store information output unit 227.
Fig. 19 is a diagram for describing an operation of the food information providing system.

### Description of Embodiments

### <Outline of System>

Hereinafter, a food information providing system according to an embodiment of the present invention will be described. Food information is information regarding a food. The food includes an ingredient, a foodstuff, a meal plan, a product, and the like. The ingredient refers to one that is consumed as it is or serves as a raw material of the foodstuff. The foodstuff refers to one obtained by processing a single or a plurality of ingredients for the purpose of preservation or seasoning. The foodstuff includes a dish, a seasoning, and the like. The meal plan refers to a combination of a plurality of ingredients and/or foodstuffs as a meal. The product is an object for selling the ingredient, the foodstuff, the dish, and the like, and includes a service. Examples of the food information can include information regarding the ingredient (material), information regarding the product or service (a menu of a restaurant) related to the food, and information regarding the dish, such as a recipe. The food information providing system of the present embodiment provides the optimal food information to individual users. In the food information providing system of the present embodiment, a relationship between an actual measurement value of information regarding a body of the user (hereinafter, referred to as physical data) and a dietary component (a nutrient, calories, or the like) taken by the user is analyzed, and a degree to which the dietary component improves or deteriorates the physical data (hereinafter, referred to as the degree of improvement/deterioration) is obtained for each dietary component.

The physical data is a measurable value related to the body (including intellect and mental strength) of the user. Examples of the physical data may include vital data such as a weight, a body fat percentage, a muscle mass, or a blood pressure, or may be a measurement value of a human ability of the user, such as a competition time or a memory performance.

A relationship between the physical data and an intake amount of the dietary component can be analyzed by, for example, multivariate analysis. Examples of the multivariate analysis can include a single regression analysis, a multiple regression analysis, a principal component analysis, an independent component analysis, a factor analysis, a discriminant analysis, a canonical discriminant analysis, a quantification theory (Types I, II, III, and IV), a multi-group quantification Type II, a cluster analysis, a conjoint analysis, a logistic regression, a multidimensional scaling (MDS), an extended quantification Type I, an extended quantification Type II, a correspondence analysis, and a covariance structure analysis.

The degree of improvement/deterioration can be, for example, a regression coefficient such as a partial regression coefficient. It may be preferable to increase or decrease the physical data depending on a type of the physical data and a target value of the user. For example, in a case where the physical data is the weight and a target weight of the user is smaller than a current weight, a degree (negative regression coefficient) of contribution to a decrease in weight is considered improvement, and in a case where the target weight of the user is larger than the current weight, a degree (positive regression coefficient) of contribution to an increase in weight is considered improvement.

The food information providing system of the present embodiment proposes the ingredient and/or dish that improves or deteriorates the physical data for an individual user by using the degree of improvement/deterioration analyzed for the user.

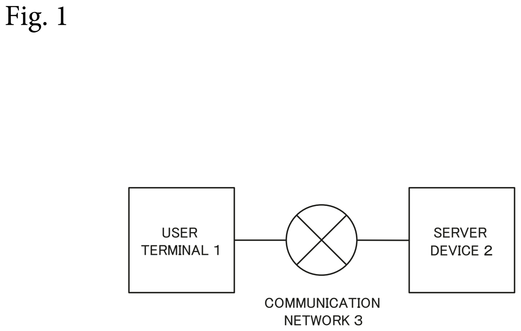

Fig. 1 is a diagram illustrating a configuration example of the food information providing system of the present embodiment. The food information providing system of the present embodiment includes a server device 2. The server device 2 is communicably connected to a user terminal 1 via a communication network 3. The communication network 3 is, for example, the Internet, and is constructed by a public telephone network, a mobile telephone network, a wireless communication path, Ethernet (registered trademark), or the like.

The user terminal 1 is a computer operated by the user, such as a smartphone, a tablet computer, or a personal computer. The user can access the server device 2 by operating the user terminal 1 to acquire the food information. A program (application) may be executed in the user terminal 1, and the application may provide a user interface to allow the user terminal 1 to access the server device 2.

The server device 2 may be, for example, a general-purpose computer such as a workstation or a personal computer, or may be logically implemented by cloud computing.

### <Server Device 2>

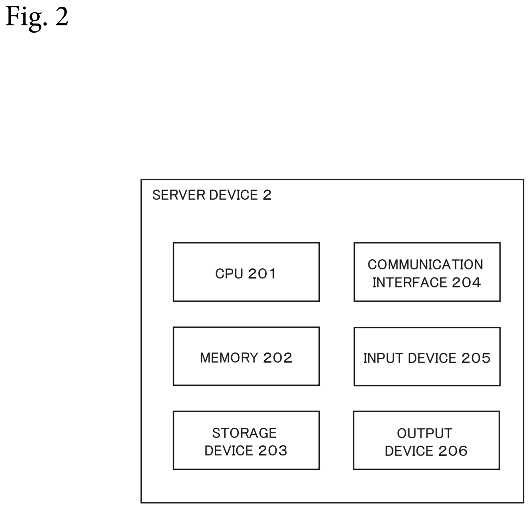

Fig. 2 is a diagram illustrating a hardware configuration example of the server device 2. The illustrated configuration is an example, and other configurations may be included. The server device 2 includes a central processing unit (CPU) 201, a memory 202, a storage device 203, a communication interface 204, an input device 205, and an output device 206. The storage device 203 is, for example, a hard disk drive, a solid state drive, a flash memory, or the like that stores various data and programs. The communication interface 204 is an interface for connection to the communication network 3, and is, for example, an adapter for connection to the Ethernet (registered trademark), a modem for connection to the public telephone line network, a wireless communication device for wireless communication, a universal serial bus (USB) connector for serial communication, or an RS232C connector. The input device 205 is, for example, a keyboard, a mouse, a touch panel, a button, or a microphone for inputting data. The output device 206 is, for example, a display, a printer, or a speaker for outputting data. Each functional unit of the server device 2 described below is implemented by the CPU 201 reading a program stored in the storage device 203 into the memory 202 and executing the program, and each storage unit of the server device 2 is implemented as a part of the memory 202 and a storage area provided by the storage device 203.

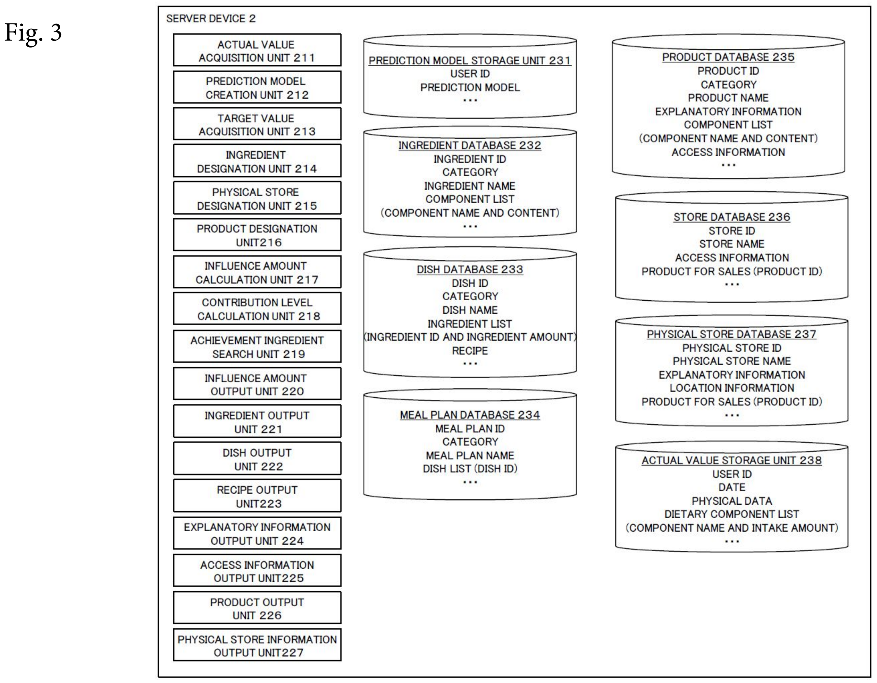

Fig. 3 is a diagram illustrating a software configuration example of the server device 2. The server device 2 includes a prediction model storage unit 231, an ingredient database 232, a dish database 233, a meal plan database 234, a product database 235, a store database 236, a physical store database 237, an actual value storage unit 238, an actual value acquisition unit 211, a prediction model creation unit 212, a target value acquisition unit 213, an ingredient designation unit 214, a physical store designation unit 215, a product designation unit 216, an influence amount calculation unit 217, a contribution level calculation unit 218, an achievement ingredient search unit 219, an influence amount output unit 220, an ingredient output unit 221, a dish output unit 222, a recipe output unit 223, an explanatory information output unit 224, an access information output unit 225, a product output unit 226, and a physical store information output unit 227.

### <Storage Unit>

The prediction model storage unit 231 stores a prediction model for predicting the physical data based on the intake amount of the dietary component. The prediction model storage unit 231 stores the prediction model for each user. That is, the prediction model storage unit 231 stores, for each user, the prediction model for predicting the physical data of the user based on intake amounts of a plurality of dietary components taken by the user. The prediction model includes the degree of improvement/deterioration for each of the dietary components. The prediction model can be a regression equation having the intake amount of the dietary component as an explanatory variable and the physical data as an objective variable, and the degree of improvement/deterioration can be a regression coefficient (for example, a partial regression coefficient). The prediction model can be created using a multiple regression analysis method. A learning model created using a machine learning method using the intake amount of the dietary component and the physical data as training data may be adopted as the prediction model.

The ingredient database 232 stores information regarding the ingredient (hereinafter, referred to as ingredient information). The ingredient information includes a content of the dietary component included in the ingredient. In the present embodiment, the ingredient information can include an ingredient ID that specifies the ingredient, a category (for example, vegetables, fishes and shellfishes, or meats) of the ingredient, an ingredient name, and the dietary component (component list) included in the ingredient. The component list can include information (component name) that specifies the dietary component and the content of one or more dietary components included in the ingredient.

The dish database 233 stores information regarding the dish (hereinafter, referred to as dish information). The dish is cooked using the ingredient, and the dish information can include a use amount of the ingredient used for the dish. In the present embodiment, the dish information can include a dish ID that specifies the dish, a category of the dish (for example, staple food, main dishes, side dishes, soups, or desserts), a dish name, one or more ingredients (ingredient list) used for the dish, and a recipe (preparation method) of the dish. The ingredient list can include the ingredient ID and the use amount (ingredient amount) of the ingredient for each ingredient used for the dish. Furthermore, the dish information may include a description (text information, image data, or the like) of the dish.

The meal plan database 234 stores information regarding the meal plan (hereinafter, referred to as meal plan information). The meal plan includes one or more dishes. The meal plan information can include information indicating the dish included in the meal plan. In the present embodiment, the meal plan information can include a meal plan ID that specifies the meal plan, a category (for example, Japanese food, western food, breakfast, dinner, or snack) of the meal plan, a meal plan name, and the dish (dish list) included in the meal plan. The dish list can include the dish ID that specifies the dish included in the meal plan.

The product database 235 stores information regarding the product to be sold (hereinafter, referred to as product information). The product can include one or more ingredients, dishes, and meal plans. The product includes the service. The product may be sold on an online store or provided in a physical store. The product information may include the content of the dietary component included in the product. In addition, the product information can include explanatory information for the product. The product information can also include access information for accessing the online store or the physical store for purchasing the product. The access information is, for example, a URL in the case of the online store, or an address or map information in the case of the physical store. In the present embodiment, the product information can include a product ID that specifies the product, a category of the product, a name of the product (product name), the explanatory information (which may be a text or may include an image) for describing the product, the dietary component (component list) included in the product, the access information for purchasing the product, a sales price of the product, and a history of the product. The component list can include information (component name) that specifies the dietary component and the content of one or more dietary components included in the product. The product history can include information related to a supply chain including processing and distribution from raw materials to final product sales, such as a raw material production area, a raw material producer, a manufacturing process, or a manufacturer, which is useful for determination of product purchase by the user based on safety and unique values (religion or creed, consideration for environment related to foodstuff manufacturing, or the like).

The store database 236 stores information regarding the online store (such as an EC site) that sells the product (hereinafter, referred to as store information). In the online store, the foodstuff can be sold through online shopping. The store information can include the access information for accessing the online store. In the present embodiment, the store information may include a store ID that specifies the online store, a name (store name) of the online store, information for accessing the online store (the access information such as the URL), and the product (product for sale) sold at the online store. The product for sale may include information (product ID) that specifies one or more products, and may include a large number of product IDs.

The physical store database 237 stores information regarding the physical store that sells the product (hereinafter, referred to as physical store information). The physical store information includes information indicating the product to be sold at the physical store. Furthermore, the physical store information may include explanatory information (which may be text data or may include image data) regarding the physical store. In the present embodiment, the physical store information can include a physical store ID that specifies the physical store, a name (physical store name) of the physical store, the explanatory information regarding the physical store, location information of the physical store (which may be, for example, latitude and longitude or the address), and the product (product for sale) sold at the physical store. The product for sale may include information (product ID) that specifies one or more products, and may include a large number of product IDs.

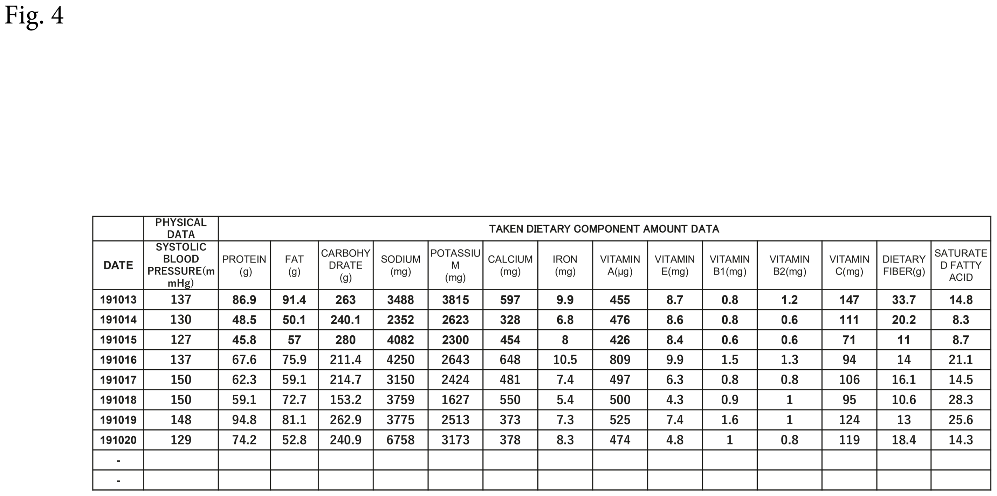

The actual value storage unit 238 stores information including the physical data of the user and an actual value of the intake amount of the dietary component (hereinafter, referred to as actual value information). The actual value information can include a user ID indicating the user, a date, an actual value (measurement value) of the physical data, and the taken dietary component (dietary component list). The dietary component list includes information (component name) that specifies the dietary component and the intake amount for each of the dietary components taken by the user. Fig. 4 is a diagram illustrating an example of the actual value information. The example in Fig. 4 illustrates an example of data for a specific user, and the date and the intake amount of the dietary component (proteins, lipids, carbohydrates, or the like) are associated with each other to form one record (actual value information).

### <Functional Unit>

The actual value acquisition unit 211 acquires the actual value of the physical data of the user. The actual value acquisition unit 211 also acquires the actual value of the intake amount of the dietary component taken by the user. The actual value acquisition unit 211 can acquire the measurement value from, for example, a physical data measurement device (not illustrated, for example, a sphygmomanometer or a scale can be used). For example, the recorded physical data can be acquired from an application that automatically records the measurement value of the physical data and is executed by the user terminal 1 or a server with which the application communicates, and can be recorded. Furthermore, the actual value acquisition unit 211 may receive the measurement value of the physical data from the user. Furthermore, for example, the actual value acquisition unit 211 can acquire an estimated intake amount of a nutrient (dietary component) from an application that estimates the intake amount of the nutrient included in the meal and is executed by the user terminal 1 or a server with which the application communicates, based on image data obtained by imaging the meal, and record the estimated intake amount. In addition, the actual value acquisition unit 211 may receive the taken dietary component from the user.

Furthermore, the actual value acquisition unit 211 may receive designation of the taken dish from the user, read the dish information corresponding to the received dish from the dish database 233, read the corresponding ingredient information from the ingredient database 232 for each ingredient included in the ingredient list included in the read dish information, and set the dietary component and the content of the read ingredient information as the actual value information.

Furthermore, the actual value acquisition unit 211 may receive designation of the meal plan taken from the user, read the meal plan information corresponding to the received meal plan from the meal plan database 234, read the dish information corresponding to each dish ID included in the dish list of the read meal plan information from the dish database 233, read the corresponding ingredient information from the ingredient database 232 for each ingredient included in the ingredient list included in the read dish information, and set the dietary component and the content of the read ingredient information as the actual value information.

The prediction model creation unit 212 creates the prediction model based on the actual value information. For example, the prediction model creation unit 212 can create the prediction model using the multiple regression analysis method with the physical data as an objective variable and the intake amount of the dietary component as an explanatory variable. The prediction model creation unit 212 can also create the prediction model using the machine learning method with the intake amount of the dietary component as input data and the physical data as supervised data.

The target value acquisition unit 213 acquires a target value of the physical data. The target value acquisition unit 213 can receive the target value from the user (receive the target value from the user terminal 1).

The ingredient designation unit 214 can receive designation of the ingredient. The ingredient designation unit 214 can receive the ingredient ID from the user (receive the ingredient ID from the user terminal 1).

The physical store designation unit 215 can receive designation of the physical store. For example, the user terminal 1 can acquire a list of the physical store information from the server device 2, display a list of the physical store names and the like, or display a location of the physical store on a map based on the location information, receive the designation of the physical store, and transmit the physical store ID indicating the designated physical store to the server device 2, and the physical store designation unit 215 can receive the physical store ID.

The product designation unit 216 can receive designation of the product. For example, the user terminal 1 reads identification information (product ID) indicating the encoded product from a code displayed on the product, and transmits the read identification information to the server device 2, and the product designation unit 216 can receive the identification information. In this case, the ingredient designation unit 214 may read the product information corresponding to the received product ID from the product database 235 and receive the content of the dietary component included in the component list of the read product information.

The influence amount calculation unit 217 calculates an amount by which the food improves or deteriorates the physical data of the user (hereinafter, referred to as an influence amount).

The influence amount calculation unit 217 can calculate the influence amount for the dietary component included in the food by the following expression. Influence amount = Coefficient a × Dietary component amount A + Coefficient b × Dietary component amount B +... + Coefficient n × Dietary component amount N = Σ(Coefficient i × Dietary component amount i)

Here, the coefficient can be a regression coefficient of the dietary component included in the prediction model. In a case where the dietary component as the explanatory variable included in the prediction model is not included in the food, the dietary component amount = 0.

The influence amount is also referred to as a "contribution amount" in a case where the food contributes to improvement, and is also referred to as an "interference amount" in a case where the food contributes to deterioration. In addition, the ingredient that contributes to improvement or deterioration but may reverse an effect thereof when taken in an amount equal to or larger than a certain amount may be determined as "exception" that is neither the contribution amount nor the interference amount.

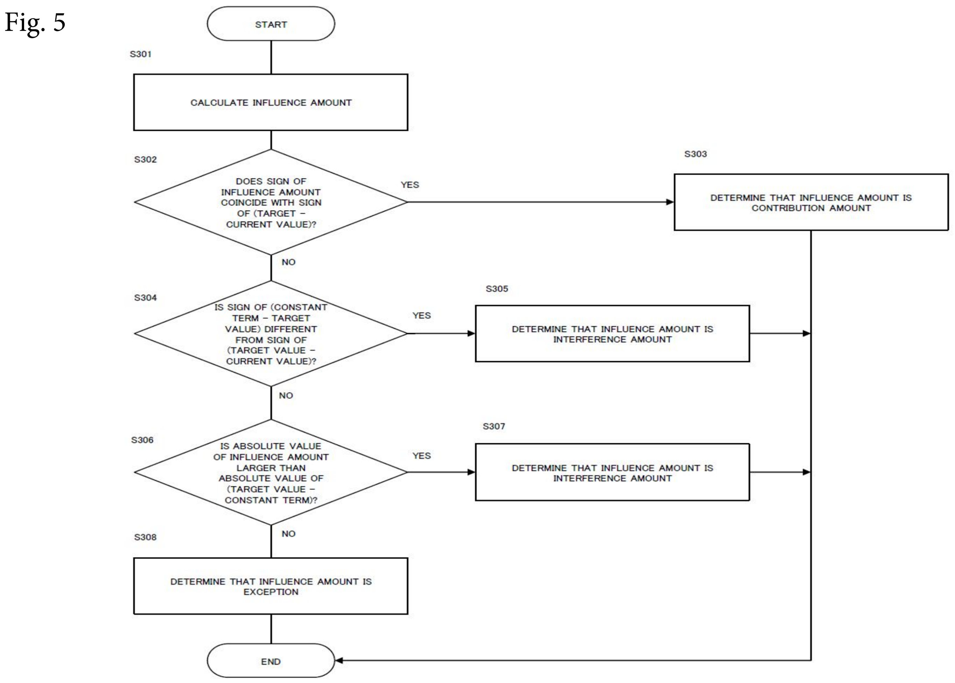

Fig. 5 is a diagram illustrating a flow of determination processing for the contribution amount and the interference amount.

The influence amount calculation unit 217 calculates the influence amount by Expression (1) (S301), and in a case where a sign of the influence amount coincides with a sign of a value obtained by subtracting a current value (for example, the latest actual value) of the physical data from the target value of the physical data (S302: YES), the influence amount calculation unit 217 determines that the influence amount is the "contribution amount" (S303).

In a case where a sign of a value obtained by subtracting the target value from a constant term of the prediction model is different from the sign of the value obtained by subtracting the current value from the target value (S304: YES), the influence amount calculation unit 217 can determine that the influence amount is the "interference amount" (S305).

In a case where an absolute value of the influence amount is larger than an absolute value of a value obtained by subtracting the constant term from the target value (S306: YES), the influence amount calculation unit 217 can determine that the influence amount is the "interference amount" (S307).

In a case where none of S302, S304, and S306 is applicable (S306: NO), the influence amount calculation unit 217 can determine that the influence amount is "exception".

In a case where the influence amount is periodically updated, a direction of the improvement is reversed when the physical data exceeds the target value, but such reversal may be prevented. For example, in a case where the weight of the user who has started to use the system for the purpose of weight loss decreases beyond the target value, it is possible to continuously reduce the weight instead of increasing the weight. In this case, in step S302 and step S304 in Fig. 5, a value to be subtracted from the target value of the physical data can be the physical data at the start of use instead of the current value of the physical data. The physical data at the start of use can be the oldest physical data corresponding to the user registered in the actual value storage unit 238. The physical data at the start of use may be stored together with the prediction model in association with the user ID in the prediction model storage unit 231, or a physical data storage unit that stores the physical data at the start of use may be provided.

### ==Influence Amount of Ingredient==

The influence amount calculation unit 217 can calculate the influence amount based on the content of the dietary component included in the ingredient and the degree of improvement/deterioration related to the dietary component (the regression coefficient in the present embodiment). The influence amount calculation unit 217 can calculate the influence amount related to the ingredient by summing values obtained by multiplying the regression coefficients by the contents.

For example, it is assumed that the prediction model is represented by the following expression. Body weight at wake-up (kg) = 0.0424 × Dietary fiber (g) -0.00176 × Calcium (mg) -0.0362 × Zinc (mg) -0.819 × Vitamin B1 (mg) + 0.683× Vitamin B2 (mg) + 0.34 × Vitamin B6 (mg) -0.015 × Saturated fatty acid (g) + 76.29

Here, when dietary component amounts (per 100 g) of "edamame", which is an ingredient, are dietary fiber = 5.0 g, calcium = 58 mg, zinc = 1.4 mg, vitamin B1= 0.31 mg, vitamin B2 = 0.15 mg, vitamin B6 = 0.15 mg, and saturated fatty acid = 0.84 g, the influence amount of "edamame" is - 0.05. In a case where weight loss is targeted, "edamame" is an improvement ingredient.

In addition, when dietary component amounts (per 100 g) of "asparagus", which is an ingredient, are dietary fiber = 1.8 g, calcium = 19 mg, zinc = 0.5 mg, vitamin B1 = 0.14 mg, vitamin B2 = 0.15 mg, vitamin B6 = 0.12 mg, and saturated fatty acid = 0.07 g, the influence amount of "asparagus" is +0.05. In a case where weight loss is targeted, "asparagus" is a deterioration ingredient.

Furthermore, in a case where the ingredient designation unit 214 receives the designation of the ingredient, the influence amount calculation unit 217 may calculate the influence amount for the received ingredient.

The influence amount output unit 220 can output the influence amount calculated by the influence amount calculation unit 217. The influence amount output unit 220 may output the influence amount for all the ingredients. The influence amount output unit 220 may output the influence amount for all the dishes. The influence amount output unit 220 may output the influence amount for all the meal plans. The influence amount output unit 220 may output the influence amount only for the designated ingredient. The influence amount output unit 220 may output the influence amount only for the designated dish. The influence amount output unit 220 may output the influence amount only for the designated product. The influence amount output unit 220 may output the influence amount only for the product sold at the designated physical store. The influence amount output unit 220 may output a total value (for example, an average value) of the dietary component amounts for a plurality of ingredients, products, and dishes.

### ==Display of Ranking=

The influence amount calculation unit 217 can calculate the influence amount for all the ingredients registered in the ingredient database 232. Furthermore, the influence amount calculation unit 217 can also determine the contribution amount, the interference amount, and the exception for the ingredient.

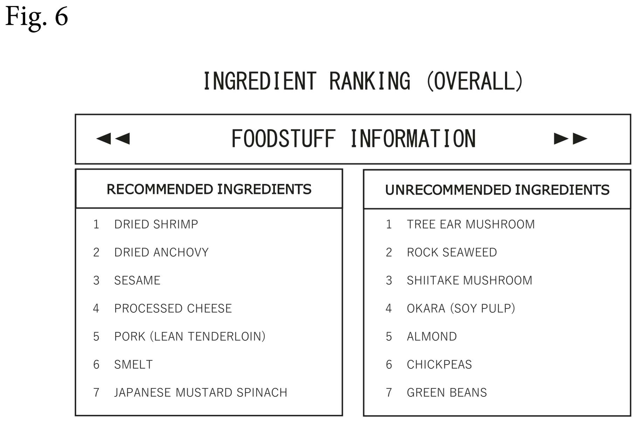

The ingredient output unit 221 can output information regarding a predetermined number of ingredients in the order of the magnitude of the influence amount. With respect to the contribution amount and/or the interference amount, the ingredient output unit 221 can also output the information regarding the predetermined number of ingredients in the order of a value or an absolute value of the contribution amount and/or the interference amount. In a case where it is determined the ingredient is the exception, the ingredient can be excluded from rankings. Fig. 6 is a diagram illustrating an output example of the ingredient output unit 221. In the example of Fig. 6, the improvement ingredient (recommended ingredient) and the deterioration ingredient (unrecommended ingredient) are output. The ingredient output unit 221 can determine that the ingredient whose influence amount is determined to be the "contribution amount" by the influence amount calculation unit 217 is the improvement ingredient, determine that the ingredient whose influence amount is determined to be the "interference amount" is the deterioration ingredient, and determine that the ingredient whose influence amount is determined to be the "exception" is not included in the improvement ingredient and the deterioration ingredient. The ingredient output unit 221 can display the ingredient names of the improvement ingredients (recommended ingredients) in descending order of the absolute value of the contribution amount, and can display the ingredient names of the deterioration ingredients (unrecommended ingredients) in descending order of the absolute value of the interference amount. In a case where it is determined that the influence amount is the exception, the ingredient output unit 221 can prevent the ingredient from being included in the rankings.

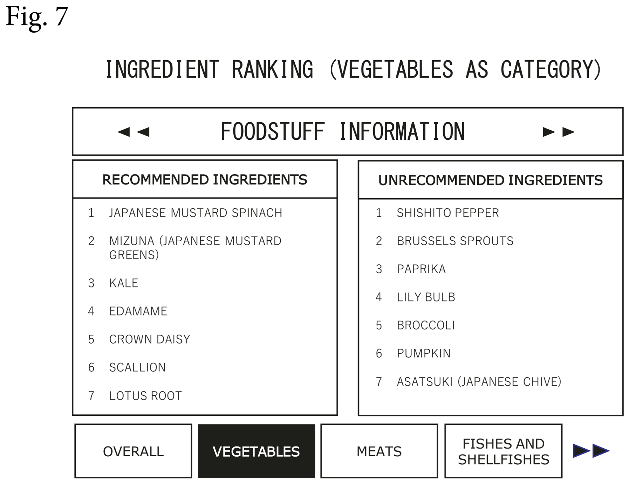

The ingredient output unit 221 can also output the information regarding the ingredients in the order of the value or the absolute value of the contribution amount and/or the interference amount for each category. Fig. 7 is a diagram illustrating an output example of the ingredient output unit 221. In the example of Fig. 7, for the improvement ingredient and the deterioration ingredient in a category of "vegetables", the ingredient names are displayed in descending order of the absolute value of the contribution amount for the improvement ingredient and in descending order of the absolute value of the interference amount for the deterioration ingredient, similarly to Fig. 6.

### ==Influence Amount of Dish==

The influence amount calculation unit 217 can also calculate the influence amount for the dish. Furthermore, the influence amount calculation unit 217 can also determine the contribution amount, the interference amount, and the exception for the dish. The influence amount calculation unit 217 can specify the content of the dietary component included in the dish from the dish database 233 and the ingredient database 232, and calculate the influence amount (and the contribution amount and the interference amount) related to the dish based on the specified content of the dietary component and the degree of improvement/deterioration (regression coefficient) related to the dietary component. The influence amount calculation unit 217 can calculate the influence amount for all the dishes registered in the dish database 233.

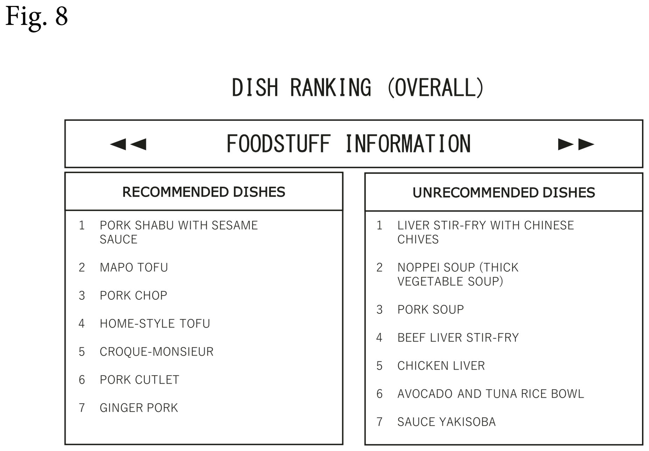

The dish output unit 222 can output information regarding a predetermined number of dishes in the order of the influence amount. The dish output unit 222 can also output the information regarding the predetermined number of dishes in the order of the value or the absolute value of the contribution amount and/or the interference amount. In a case where it is determined the dish is the exception, the dish can be excluded from rankings. The dish output unit 222 can determine that the dish whose influence amount is determined to be the "contribution amount" by the influence amount calculation unit 217 is an improvement dish, determine that the dish whose influence amount is determined to be the "interference amount" is a deterioration dish, and determine that the dish whose influence amount is determined to be the "exception" is not included in the improvement dish or the deterioration dish. Fig. 8 is a diagram illustrating an output example of the dish output unit 222. In the example of Fig. 8, the dish names of the improvement dishes (recommended dishes) are displayed in descending order of the absolute value of the contribution amount, and the dish names of the deterioration dishes (unrecommended dishes) are displayed in descending order of the absolute value of the interference amount.

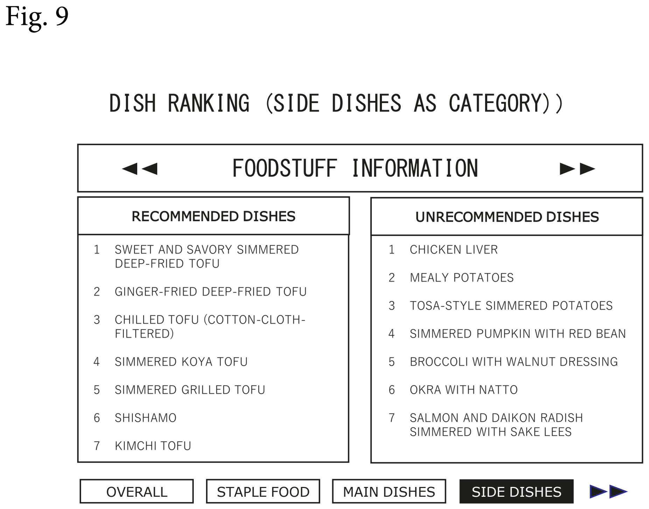

The dish output unit 222 can also output the information regarding the dishes in the order of the value or the absolute value of the influence amount, the contribution amount, and/or the interference amount for each category. Fig. 9 is a diagram illustrating an output example of the dish output unit 222. In the example of Fig. 9, for the improvement dish and the deterioration dish in a category of "side dishes", the dish names are displayed in descending order of the absolute value of the contribution amount and the absolute value of the interference amount as in Fig. 8.

### ==Influence Amount of Product==

The influence amount calculation unit 217 can also calculate the influence amount for the product. In addition, the influence amount calculation unit 217 can also determine the contribution amount, the interference amount, and the exception for the product. In a case where the product designation unit 216 receives the designation of the product, the influence amount calculation unit 217 can also calculate the influence amount related to the received product. The influence amount calculation unit 217 can read the product information corresponding to the product ID received by the product designation unit 216 from the product database 235, and can calculate a degree (the influence amount of the product) to which the product improves or deteriorates the physical data based on the content and the degree of improvement/deterioration for each dietary component included in the component list of the read product information (for example, the sum of values obtained by multiplying the contents by the regression coefficients).

The product output unit 226 can output information regarding a predetermined number of products in the order of the magnitude of the influence amount. The product output unit 226 can also output the information regarding the predetermined number of products in the order of the value or the absolute value of the contribution amount and/or the interference amount. In a case where it is determined the product is the exception, the product can be excluded from rankings. The ingredient output unit 221 can determine that the product whose influence amount is determined to be the "contribution amount" by the influence amount calculation unit 217 is an improvement product, determine that the product whose influence amount is determined to be the "interference amount" is a deterioration product, and determine that the product whose influence amount is determined to be the "exception" is not included in the improvement product and the deterioration product. Similarly to the ingredient illustrated in Fig. 6 and the dish illustrated in Fig. 8, the product output unit 226 can display the product names of the improvement products (recommended products) in descending order of the absolute value of the contribution amount, and can display the product names of the deterioration products (unrecommended products) in descending order of the absolute value of the interference amount.

The product output unit 226 can also output the information regarding the products in the order of the value or the absolute value of the contribution amount and/or the interference amount for each category. In this case, similarly to the ingredient illustrated in Fig. 7 and the dish illustrated in Fig. 9, the product output unit 216 can display the product names of the improvement products and the deterioration products in a specific category in the order of the value or the absolute value of the contribution amount and/or the interference amount.

### ==Influence Amount of Product Sold at Physical Store==

In a case where the physical store designation unit 215 receives the designation of the physical store, the influence amount calculation unit 217 can calculate the influence amount (and the contribution amount and the interference amount) for the product sold at the received physical store. The influence amount calculation unit 217 can read the physical store information corresponding to the physical store ID received by the physical store designation unit 215 from the physical store database 237, read the product information corresponding to the product ID from the product database 235 for each of the product IDs included in the products for sale of the read physical store information, and calculate the influence amount of the product based on the content and the degree of improvement/deterioration for each dietary component included in the component list of the read product information.

The product output unit 226 can display the information (for example, the product name) regarding the product in the order of the value or the absolute value of the contribution amount and/or the interference amount only for the product sold at the designated physical store. The influence amount output unit 220 (or the product output unit 226) can output the influence amount (the contribution amount and/or the interference amount) in association with each product.

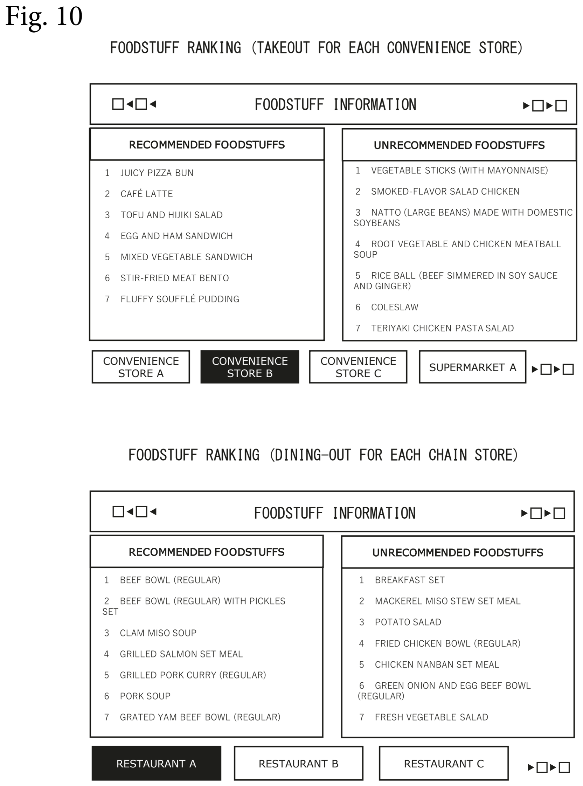

Fig. 10 is a diagram illustrating an output example of the product output unit 226. In the upper part of Fig. 10, in a case where the physical store (convenience store B) is designated, the products sold at the physical store are ranked and displayed according to the values or the absolute values of the contribution amount and the interference amount. In the lower part of Fig. 10, in a case where the physical store (restaurant A) is designated, the products provided at the physical store are ranked and displayed according to the values or the absolute values of the contribution amount and the interference amount.

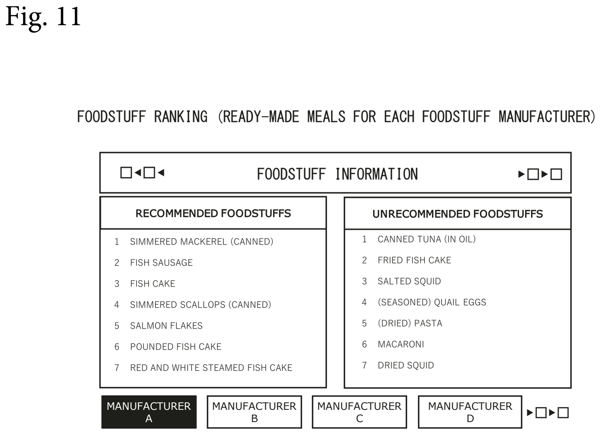

The product output unit 226 can also narrow down and output the products. Fig. 11 is a diagram illustrating an output example of the product output unit 226. In the example of Fig. 11, after narrowing the products down to products of a specific manufacturer (a manufacturer can be set in the category of the product information, and a category including the designated manufacturer can be searched), the products can be ranked and displayed according to the value or the absolute value of the contribution amount and/or the interference amount.

### ==Contribution Level==

The contribution level calculation unit 218 calculates a degree to which contribution is made to target achievement related to the physical data (hereinafter, referred to as a contribution level). The contribution level can be a ratio of a difference between a constant of the regression equation and the target value with respect to the influence amount. That is, the contribution level calculation unit 218 can calculate the contribution level by the following expression. Contribution level = {Influence amount ÷ (Target value - Constant term of prediction model)} × 100

In display of the rankings of the ingredients, dishes, products, and the like described above, the contribution level may be used instead of the contribution amount and/or the interference amount. It is sufficient if the contribution level calculated based on the contribution amount is rephrased as the degree of contribution or the contribution amount, and the contribution level calculated based on the interference amount is rephrased as the degree of interference or the interference amount.

If the foodstuff is taken such that the sum of the degree of contribution and the degree of interference becomes 100, the amounts of the taken dietary components enable the target achievement. In this way, it is also possible to determine the intake amount of the foodstuff.

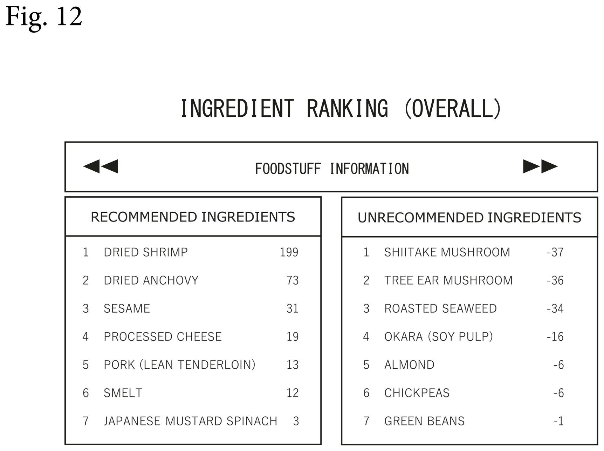

The ingredient output unit 221 can display the contribution level by including the contribution level in the information regarding the ingredient. Fig. 12 is a diagram illustrating an output example of the ingredient output unit 221. In the example of Fig. 12, the contribution level is displayed together with the product name. Furthermore, in the example of Fig. 12, the ingredient whose degree of contribution is high is indicated as the "recommended ingredient", and the ingredient whose degree of interference is low (the absolute value is small) is indicated as the "unrecommended ingredient".

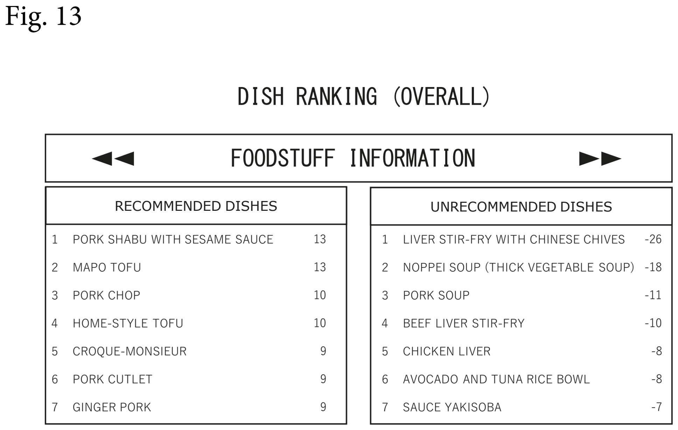

The dish output unit 222 can display the contribution level by including the contribution level in the information regarding the dish. Fig. 13 is a diagram illustrating an output example of the dish output unit 222. In the example of Fig. 13, the contribution level is displayed together with the dish name. Also, in the example of Fig. 13, the dish whose degree of contribution is high is indicated as the "recommended dish", and the dish whose degree of interference is low (the absolute value is large) is indicated as the "unrecommended dish".

### ==Proposal for Target Achievement==

The achievement ingredient search unit 219 searches for the ingredient for achieving the target of the physical data. The achievement ingredient search unit 219 can search the ingredient database 232 for one or more ingredients including the dietary component necessary for making the actual value be equal to the target value or making the actual value exceed the target value based on a difference between the target value and the actual value and the influence amount. For example, the achievement ingredient search unit 219 can search for a predetermined number of improvement ingredients in descending order of the contribution amount or the degree of contribution. A proposal for improvement beyond the target may also be provided. For example, a case where the actual value exceeds the target value when the contribution amount is added to the constant term of the prediction model may also be allowed.

The ingredient output unit 221 can output the information (for example, the ingredient name) regarding the ingredient searched by the achievement ingredient search unit 219.

Although the ingredient output unit 221 described above outputs the ingredient name, the ingredient output unit 221 may output the explanatory information by including the explanatory information in the ingredient information, may search the product database 235 for the product including the ingredient and output one or more items included in the searched product information, or may search the physical store database 237 or the store database 236 for the physical store or the online store that sells the searched product and output the access information or the location information of the searched physical store or online store.

### ==Output of Recipe==

The recipe output unit 223 can output the recipe of the dish using the designated ingredient. For example, the recipe output unit 223 can search the dish database 233 for the dish information including the ingredient ID received by the ingredient designation unit 214 and output the recipe included in the searched dish information. The recipe output unit 223 can output the recipe for one or more dishes in descending order of the absolute value of the contribution amount or the degree of contribution of the dish in the searched dish information. In addition, the recipe output unit 223 may receive the designation of the dish (an input of the dish ID) and output the recipe of the dish information corresponding to the designated dish ID.

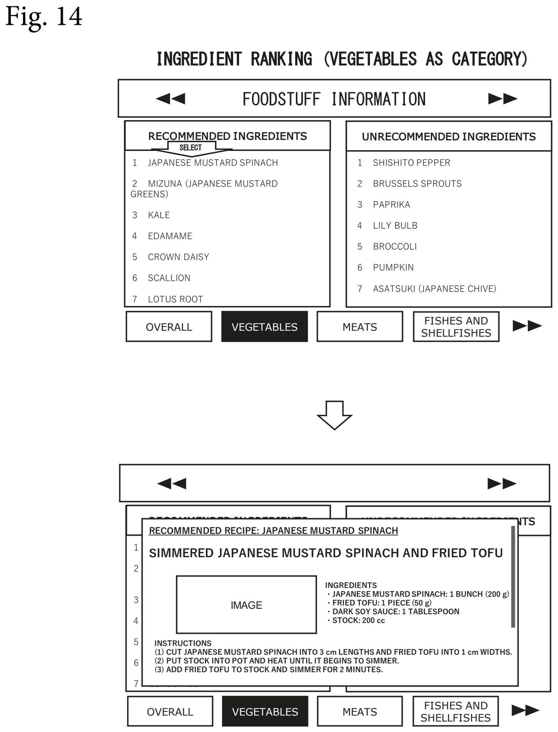

Fig. 14 is a diagram illustrating an output example of the recipe output unit 223. The example of Fig. 14 illustrates a state in which the recipe of the dish which has the largest absolute value of the contribution amount or the degree of contribution and uses "Japanese mustard spinach" is displayed in a case where the ingredient "Japanese mustard spinach" is selected.

### ==Output of Description of Product==

The explanatory information output unit 224 outputs the explanatory information regarding the product. The explanatory information output unit 224 can read the product information corresponding to the product ID received by the product designation unit 216 from the product database 235 and output the explanatory information included in the read product information.

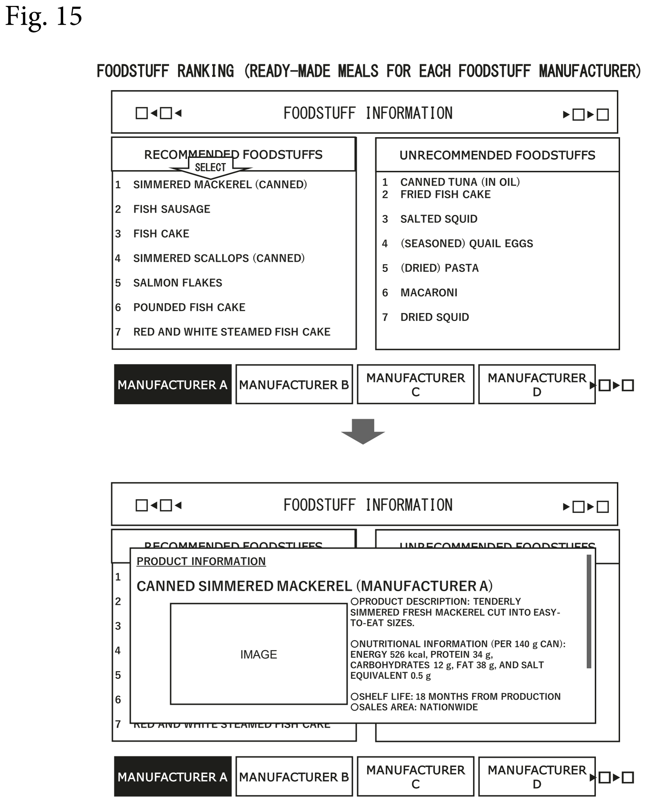

Fig. 15 is a diagram illustrating an output example of the explanatory information output unit 224. The example of Fig. 15 illustrates a state in which the explanatory information regarding the product is displayed in a case where a product "canned simmered mackerel (manufacturer A)" is selected.

### ==Output of Store/Physical Store==

The access information output unit 225 outputs the access information for accessing the online store where the designated product can be purchased. The access information output unit 225 can read the product information corresponding to the product ID received by the product designation unit 216 from the product database 235 and output the access information included in the read product information. The access information is, for example, a URL.

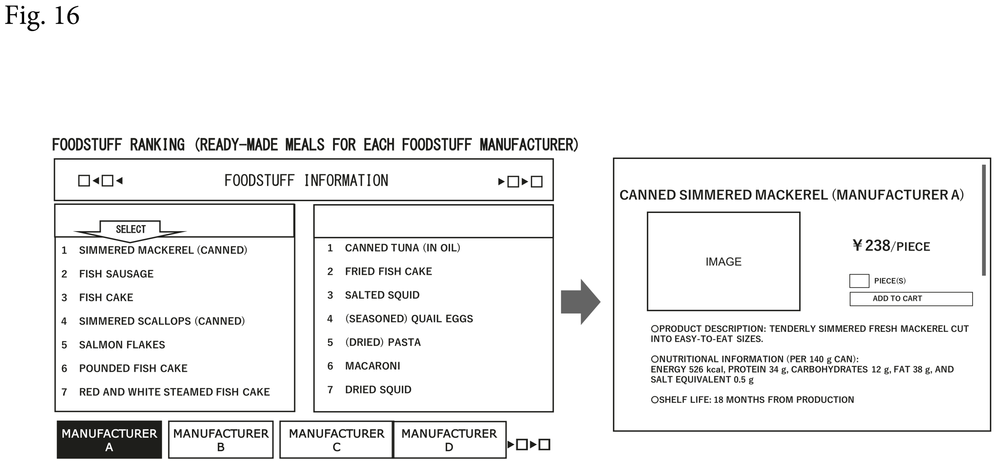

Fig. 16 is a diagram illustrating an example in which the access information is output by the access information output unit 225. In the example of Fig. 16, a screen example in which the access information such as the URL for accessing the online store such as the EC site where the product can be purchased is displayed in a case where the product "simmered mackerel (canned)" is selected, and the user terminal 1 accesses the online store according to the access information is illustrated. The product can be purchased by general online shopping processing on a screen of the online store as illustrated in the right part of Fig. 16.

The physical store information output unit 227 can output the information regarding the physical store where the designated product can be purchased. The physical store information output unit 227 can search the physical store database 237 for the physical store information including the product ID received by the product designation unit 216 in the product for sale, and output the physical store name, the explanatory information, the location information, and the like included in the searched physical store information.

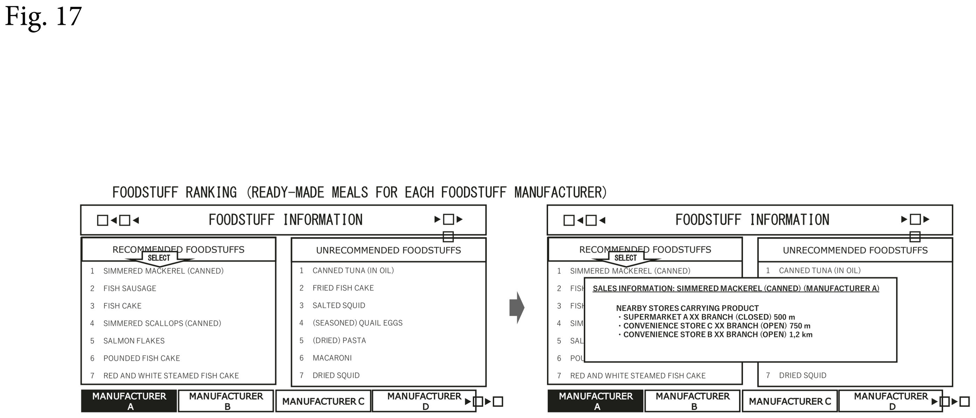

Fig. 17 is a diagram illustrating an output example of the physical store information output unit 227. In the example of Fig. 17, in a case where the product "simmered mackerel (canned)" is selected as in the left part, the name of the physical store that sells the product is displayed as in the right part. The physical store information output unit 227 can acquire information regarding a current location from the user terminal 1, calculate a distance from the acquired current location to the location information of the physical store information including the designated product ID, and output the physical store names of a predetermined number (three in the example of Fig. 17) of physical stores and the distances to the physical stores in ascending order of proximity. Furthermore, the physical store information may include business hours, and as illustrated in Fig. 17, the physical store information output unit 227 may also output information indicating whether or not a current time is within the business hours.

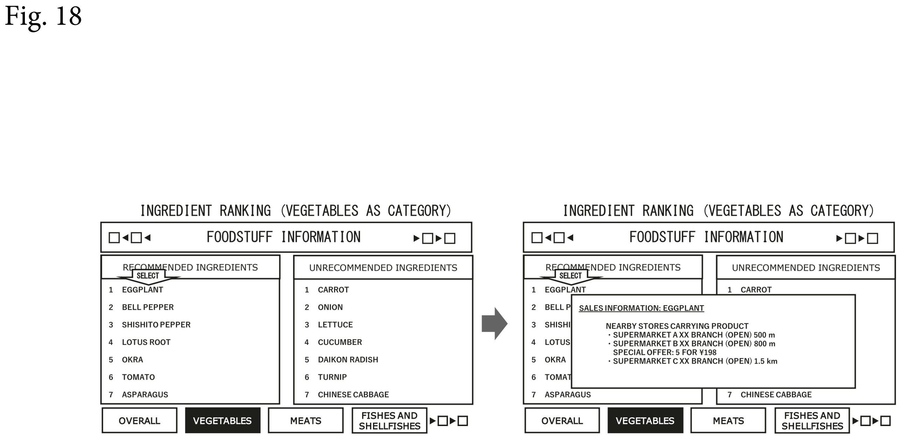

The physical store information output unit 227 can also output the physical store that sells the ingredient when the designation of the ingredient is received. In this case, for example, in the physical store that sells the ingredient as the product, such as a supermarket, the ingredient ID can be included in the product for sale in the physical store information. In addition, the product ID or ingredient ID and a sales price thereof may be included in the product for sale in the physical store information, and the sales price of each physical store may be displayed together. Fig. 18 is a diagram illustrating an output example of the physical store information output unit 227. In the example of Fig. 18, in a case where an ingredient "eggplant" is selected, the physical store information regarding the physical store (supermarket) that sells "eggplant" is illustrated in the right part.

### <Operation>

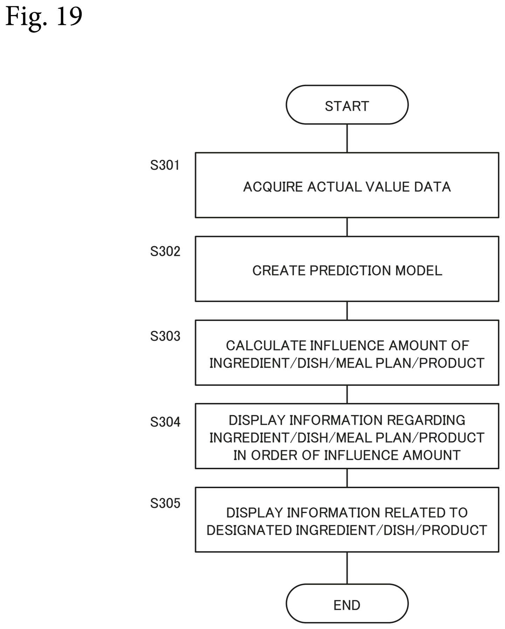

Fig. 19 is a diagram for describing an operation of the food information providing system.

The server device 2 acquires the physical data of the user and actual value data related to the intake amount of the dietary component (S301), and creates the prediction model for predicting the physical data from the intake amount of the dietary component based on the actual value data (S302).

The server device 2 calculates the influence amount of the ingredient on the improvement and deterioration of the physical data, the influence amount of the dish using the ingredient on the improvement and deterioration of the physical data, the influence amount of the meal plan including the dish on the improvement and deterioration of the physical data, and/or the influence amount of the product including the dietary component on the improvement and deterioration of the physical data (S303), displays information regarding the ingredient, the dish, the meal plan, and/or the product in the order of the influence amount (S304), and outputs the information regarding the designated ingredient, dish, and/or product (the names of the ingredient, the dish, and the product, the explanatory information, the information regarding the online store and the physical store where the ingredient, dish, and/or product is sold, a price of the product, a history of the product, and the like) in a case where the designation of the ingredient, dish, and/or product is received from the user (S305). The ingredients, dishes, meal plans, and/or products may be displayed in the order of the contribution level by calculating the contribution level in addition to the influence amount.

As described above, with the food information providing system of the present embodiment, it is possible to output the information regarding the ingredient, the dish, the meal plan, and the product that affect the improvement and/or deterioration of the physical data. Therefore, since the user can easily select the ingredient, the dish, the meal plan, and the product effective for improving the physical data, the physical data is expected to achieve the target value.

Furthermore, with the food information providing system of the present embodiment, for example, it is also possible to create a daily meal plan for achieving the target by combining the dishes. In the case of assuming a realistic dietary life, it is conceivable that a daily meal plan includes a combination of the dish and the ingredient (tomato, banana, or the like) that can be taken as it is, and the product (the ingredient, the foodstuff, and the meal plan sold by a business operator) may be partially combined. In this way, based on the target achievement using the prediction model, in the dietary life of the user, the food to be taken by the user can be designed by the user or designed automatically according to determination criteria such as a preference, an occasion (breakfast, lunch, dinner, or snack/late-night meal), costs (economical, routine, or luxurious), a method (home cooking, takeout, dining-out, or delivery), and values, and the user can be prompted to cook or purchase the food.

Although the present embodiment has been described above, the above embodiment is for facilitating understanding of the present invention, and is not intended to limit and interpret the present invention. The present invention can be modified and improved without departing from the gist thereof, and the present invention includes equivalents thereof.

For example, in the above-described embodiment, for the ingredient, the dish, and the product, the improvement ingredient/improvement dish/improvement product and/or the deterioration ingredient/deterioration dish/deterioration product are ranked in descending order of the absolute value of the contribution amount and/or the interference amount (or the degree of contribution and/or the degree of interference), but the ranking may be performed using the value of the influence amount or the degree of influence as it is. In this case, in a case where the target is to increase the physical data, it is sufficient if the ingredient/dish/product whose influence amount is large is determined as the improvement ingredient/improvement dish/improvement product and the ingredient/dish/product whose influence amount is small is determined as the deterioration ingredient/deterioration dish/deterioration product. As for the rankings in this case, it is sufficient if the improvement ingredients/improvement dishes/improvement products are arranged in descending order of the influence amount, and the deterioration ingredients/deterioration dishes/deterioration products are arranged in ascending order of the influence amount. On the other hand, in a case where the target is to decrease the physical data, it is sufficient if the ingredient/dish/product whose influence amount is small is determined as the improvement ingredient/improvement dish/improvement product and the ingredient/dish/product whose influence amount is large is determined as the deterioration ingredient/deterioration dish/deterioration product. As for the rankings in this case, it is sufficient if the improvement ingredients/improvement dishes/improvement products are arranged in ascending order of the influence amount, and the deterioration ingredients/deterioration dishes/deterioration products are arranged in descending order of the influence amount. In the case of using the contribution level, regardless of whether the target is to increase or to decrease the physical data, it is sufficient if the ingredient/dish/product whose contribution level is high is determined as the improvement ingredient/improvement dish/improvement product, and the ingredient/dish/product whose contribution level is low is determined as the deterioration ingredient/deterioration dish/deterioration product. As for the rankings, it is sufficient if the improvement ingredients/improvement dishes/improvement products are arranged in descending order of the contribution level, and the deterioration ingredients/deterioration dishes/deterioration products are arranged in ascending order of the contribution level.

### ==Influence Amount/Contribution Level of Meal Plan===

In addition, the present invention similarly includes calculating the influence amount or the contribution level from the amount of the dietary component included in the meal plan including one or more ingredients, dishes, and products, and ranking the improvement meal plans/deterioration meal plans.

In this case, for example, for each piece of meal plan information (all the pieces of meal plan information may be used, or only the meal plan information satisfying a search condition such as the category designated by the user may be used) registered in the meal plan database 234, the influence amount calculation unit 217 can read, from the dish database 233, the dish information corresponding to the dish ID included in the dish list, read, from the ingredient database 232, the ingredient information corresponding to the ingredient ID included in the ingredient list of the read dish information, and specify the content of the dietary component included in the meal plan based on the dietary component included in the read ingredient information and the content thereof. The influence amount calculation unit 217 can calculate the influence amount, the contribution amount, and/or the interference amount related to the meal plan based on the specified content of the dietary component and the degree of improvement/deterioration (regression coefficient) related to the dietary component.

Furthermore, the server device 2 can include a meal plan output unit. The meal plan output unit can output information regarding a predetermined number of meal plans in the order of the magnitude of the influence amount. The meal plan output unit can also output information regarding a predetermined number of meal plans in the order of the value or the absolute value of the contribution amount and/or the interference amount. The meal plan output unit can determine that the dish whose influence amount is determined to be the "contribution amount" by the influence amount calculation unit 217 is the improvement meal plan, and determine that the dish whose influence amount is determined to be the "interference amount" is the deterioration meal plan. In a case where the dish corresponding to the "exception" is included, the meal plan may be excluded. For example, the meal plan output unit can display the meal plan name and a content of the meal plan (for example, the name or the like of the included dish) in descending order of the absolute value of the contribution amount by setting the improvement meal plan as a "recommended meal plan", and can display the meal plan name and the content of the meal plan in descending order of the absolute value of the interference amount by setting the deterioration meal plan as an "unrecommended meal plan".

Furthermore, the meal plan output unit may output pieces of information regarding the meal plans in the order of the value or the absolute value of the influence amount, the contribution amount, and/or the interference amount for each category. For example, the meal plan output unit can display the meal plan name and the content of the meal plan in descending order of the absolute value of the contribution amount and the absolute value of the interference amount for the improvement meal plan and the deterioration meal plan related to the category such as "Japanese food", "Western food", or "breakfast".

### <Disclosed Items>

Note that the present disclosure also includes the following configurations.

### [Item 1]

An information processing system including:
a prediction model storage unit that stores, for each user, a prediction model that predicts physical data regarding a body of the user based on intake amounts of a plurality of dietary components taken by the user, the prediction model including a degree to which each dietary component improves or deteriorates the physical data;
a food database that stores a content of the dietary component contained in a food; and
an influence amount calculation unit that calculates an influence amount which is an amount by which the food improves or deteriorates the physical data of the user based on the content of the dietary component included in the food and the degree related to the dietary component.

### [Item 2]

The information processing system according to Item 1, further including
a food output unit that outputs a predetermined number of foods in an order of a magnitude of the influence amount or a value corresponding to the influence amount.

### [Item 3]

The information processing system according to Item 1, further including
a dish database that stores a use amount of an ingredient used for a dish, in which
the food database stores the content of the dietary component included in the ingredient, and
the influence amount calculation unit specifies the content of the dietary component included in the dish from the dish database and the food database, and calculates the influence amount based on the specified content of the dietary component and the degree related to the dietary component.

### [Item 4]

The information processing system according to Item 3, further including
a dish output unit that outputs the dish in an order of a magnitude of the influence amount or a value corresponding to the influence amount.

### [Item 5]

The information processing system according to Item 1, in which
the prediction model is a regression equation using the dietary component as an explanatory variable and the physical data as an objective variable,
the degree is a regression coefficient, and
the influence amount calculation unit calculates the influence amount by summing values obtained by multiplying the regression coefficients by the content.

### [Item 6]

The information processing system according to Item 5, further including:
a target value acquisition unit that acquires a target value of the physical data; and
a contribution/interference degree calculation unit that calculates a degree of contribution/interference that is a ratio of a difference between a constant of the regression equation and the target value with respect to the influence amount.

### [Item 7]

The information processing system according to Item 1, in which
the food database stores a category corresponding to the food, and
the information processing system further includes a food output unit that outputs the food in an order of a magnitude of the influence amount or a value corresponding to the influence amount for each category.

### [Item 8]

The information processing system according to Item 3, in which
the dish database stores a category corresponding to the dish, and
the information processing system further includes a dish output unit that outputs the dish in an order of a magnitude of the influence amount or a value corresponding to the influence amount for each category.

### [Item 9]

The information processing system according to Item 1, further including:
a dish database that stores a use amount of the food used for the dish and a recipe of the dish;
a food designation unit that receives designation of the food; and
a recipe output unit that reads the recipe of the dish using the received food from the dish database and outputs the read recipe.

### [Item 10]

The information processing system according to Item 1, further including:
a food designation unit that receives designation of the food; and
an influence amount output unit that outputs the influence amount, in which
the influence amount calculation unit calculates the influence amount for the food received by the food designation unit.

### [Item 11]

The information processing system according to Item 10, further including
a product database that stores the content of the dietary component included in a product, in which
the food designation unit reads identification information indicating the product from a code which is displayed on the product and in which the identification information is encoded, and reads the content of the dietary component corresponding to the read identification information from the product database, and
the influence amount calculation unit calculates the influence amount based on the content read from the product database and the degree.

### [Item 12]

The information processing system according to Item 1, further including:
a target value acquisition unit that acquires a target value of the physical data;
an achievement food search unit that searches the food database for one or more foods including the dietary component necessary for achieving the target value based on the prediction model; and
a food output unit that outputs the searched food.

### [Item 13]

The information processing system according to Item 1, further including:
a product database that stores the content of the dietary component included in a product;
a physical store database that stores the product sold at a physical store for each physical store;
a physical store designation unit that receives designation of the physical store; and
an influence amount output unit that outputs the influence amount, in which
the influence amount calculation unit specifies the product corresponding to the physical store received by the physical store designation unit by referring to the physical store database, and calculates the influence amount for each specified product based on the content and the degree, and
the influence amount output unit outputs the influence amount in association with the specified product.

### [Item 14]

The information processing system according to Item 13, in which
the product database further stores explanatory information regarding the product, and
the information processing system further includes:
   a product designation unit that receives designation of the product; and
   an explanatory information output unit that reads the explanatory information corresponding to the received product from the product database and outputs the explanatory information.

### [Item 15]

The information processing system according to Item 13, further including:
a store database that stores access information for accessing an online store where the product is purchased in association with the product;
a product designation unit that receives designation of the product; and
an access information output unit that reads the access information corresponding to the received product from the store database and outputs the access information.

### [Item 16]

The information processing system according to Item 1, further including:
a product database that stores the content of the dietary component included in a product; and
a physical store database that stores explanatory information regarding the product sold at a physical store and the physical store for each physical store, in which
the influence amount calculation unit calculates an influence amount which is an amount by which the product improves or deteriorates the physical data of the user based on the content of the dietary component corresponding to the product and the degree related to the dietary component, and
the information processing system further includes:
   a product output unit that outputs a predetermined number of the products in an order of a magnitude of the influence amount;
   a product designation unit that receives designation of the product; and
   a physical store information output unit that reads the explanatory information corresponding to the received product from the physical store database and outputs the explanatory information.

### [Item 17]

An information processing method executed by a computer, the information processing method including:
storing, for each user, a prediction model that predicts physical data regarding a body of the user based on intake amounts of a plurality of dietary components taken by the user, the prediction model including a degree to which each dietary component improves or deteriorates the physical data;
storing a content of the dietary component contained in a food; and
calculating an influence amount which is an amount by which the food improves or deteriorates the physical data of the user based on the content of the dietary component included in the food and the degree related to the dietary component.

### [Item 18]

A program for causing a computer to execute:
storing, for each user, a prediction model that predicts physical data regarding a body of the user based on intake amounts of a plurality of dietary components taken by the user, the prediction model including a degree to which each dietary component improves or deteriorates the physical data;
storing a content of the dietary component contained in a food; and
calculating an influence amount which is an amount by which the food improves or deteriorates the physical data of the user based on the content of the dietary component included in the food and the degree related to the dietary component.

### Reference Signs List

- 1: User terminal
- 2: Server device

## Claims

1. An information processing system comprising:
a prediction model storage unit that stores, for each user, a prediction model that predicts physical data regarding a body of the user based on intake amounts of a plurality of dietary components taken by the user, the prediction model including a degree to which each dietary component improves or deteriorates the physical data;
a food database that stores a content of the dietary component contained in a food; and
an influence amount calculation unit that calculates an influence amount which is an amount by which the food improves or deteriorates the physical data of the user based on the content of the dietary component included in the food and the degree related to the dietary component.

2. The information processing system according to claim 1, further comprising
a food output unit that outputs a predetermined number of foods in an order of a magnitude of the influence amount or a value corresponding to the influence amount.

3. The information processing system according to claim 1, further comprising
a dish database that stores a use amount of an ingredient used for a dish, wherein
the food database stores the content of the dietary component included in the ingredient, and
the influence amount calculation unit specifies the content of the dietary component included in the dish from the dish database and the food database, and calculates the influence amount based on the specified content of the dietary component and the degree related to the dietary component.

4. The information processing system according to claim 3, further comprising
a dish output unit that outputs the dish in an order of a magnitude of the influence amount or a value corresponding to the influence amount.

5. The information processing system according to claim 1, wherein
the prediction model is a regression equation using the dietary component as an explanatory variable and the physical data as an objective variable,
the degree is a regression coefficient, and
the influence amount calculation unit calculates the influence amount by summing values obtained by multiplying the regression coefficient by the content.

6. The information processing system according to claim 5, further comprising:
a target value acquisition unit that acquires a target value of the physical data; and
a contribution/interference degree calculation unit that calculates a degree of contribution/interference that is a ratio of a difference between a constant of the regression equation and the target value with respect to the influence amount.

7. The information processing system according to claim 1, wherein
the food database stores a category corresponding to the food, and
the information processing system further comprises a food output unit that outputs the food in an order of a magnitude of the influence amount or a value corresponding to the influence amount for each category.

8. The information processing system according to claim 3, wherein
the dish database stores a category corresponding to the dish, and
the information processing system further comprises a dish output unit that outputs the dish in an order of a magnitude of the influence amount or a value corresponding to the influence amount for each category.

9. The information processing system according to claim 1, further comprising:
a dish database that stores a use amount of the food used for the dish and a recipe of the dish;
a food designation unit that receives designation of the food; and
a recipe output unit that reads the recipe of the dish using the received food from the dish database and outputs the read recipe.

10. The information processing system according to claim 1, further comprising:
a food designation unit that receives designation of the food; and
an influence amount output unit that outputs the influence amount, wherein
the influence amount calculation unit calculates the influence amount for the food received by the food designation unit.

11. The information processing system according to claim 10, further comprising
a product database that stores the content of the dietary component included in a product, wherein
the food designation unit reads identification information indicating the product from a code which is displayed on the product and in which the identification information is encoded, and reads the content of the dietary component corresponding to the read identification information from the product database, and
the influence amount calculation unit calculates the influence amount based on the content read from the product database and the degree.

12. The information processing system according to claim 1, further comprising:
a target value acquisition unit that acquires a target value of the physical data;
an achievement food search unit that searches the food database for one or more foods including the dietary component necessary for achieving the target value based on the prediction model; and
a food output unit that outputs the searched food.

13. The information processing system according to claim 1, further comprising:
a product database that stores the content of the dietary component included in a product;
a physical store database that stores the product sold at a physical store for each physical store;
a physical store designation unit that receives designation of the physical store; and
an influence amount output unit that outputs the influence amount, wherein
the influence amount calculation unit specifies the product corresponding to the physical store received by the physical store designation unit by referring to the physical store database, and calculates the influence amount for each specified product based on the content and the degree, and
the influence amount output unit outputs the influence amount in association with the specified product.

14. The information processing system according to claim 13, wherein
the product database further stores explanatory information regarding the product, and
the information processing system further comprises:
a product designation unit that receives designation of the product; and
an explanatory information output unit that reads the explanatory information corresponding to the received product from the product database and outputs the explanatory information.

15. The information processing system according to claim 13, further comprising:
a store database that stores access information for accessing an online store where the product is purchased in association with the product;
a product designation unit that receives designation of the product; and
an access information output unit that reads the access information corresponding to the received product from the store database and outputs the access information.

16. The information processing system according to claim 1, further comprising:
a product database that stores the content of the dietary component included in a product; and
a physical store database that stores explanatory information regarding the product sold at a physical store and the physical store for each physical store, wherein
the influence amount calculation unit calculates an influence amount which is an amount by which the product improves or deteriorates the physical data of the user based on the content of the dietary component corresponding to the product and the degree related to the dietary component, and
the information processing system further comprises:
a product output unit that outputs a predetermined number of the products in an order of a magnitude of the influence amount;
a product designation unit that receives designation of the product; and
a physical store information output unit that reads the explanatory information corresponding to the received product from the physical store database and outputs the explanatory information.

17. An information processing method executed by a computer, the information processing method comprising:
storing, for each user, a prediction model that predicts physical data regarding a body of the user based on intake amounts of a plurality of dietary components taken by the user, the prediction model including a degree to which each dietary component improves or deteriorates the physical data;
storing a content of the dietary component contained in a food; and
calculating an influence amount which is an amount by which the food improves or deteriorates the physical data of the user based on the content of the dietary component included in the food and the degree related to the dietary component.

18. A program for causing a computer to execute:
storing, for each user, a prediction model that predicts physical data regarding a body of the user based on intake amounts of a plurality of dietary components taken by the user, the prediction model including a degree to which each dietary component improves or deteriorates the physical data;
storing a content of the dietary component contained in a food; and
calculating an influence amount which is an amount by which the food improves or deteriorates the physical data of the user based on the content of the dietary component included in the food and the degree related to the dietary component.
